(19) Europäisches Patentamt
European Patent Office
Office européen des brevets

(11) EP 3 269 747 B1

(12) EUROPEAN PATENT SPECIFICATION

(45) Date of publication and mention of the grant of the patent:
**20.10.2021 Bulletin 2021/42**

(21) Application number: **16761755.4**

(22) Date of filing: **08.03.2016**

(51) Int Cl.:
*A61L 31/10* (2006.01) *A61L 31/16* (2006.01)
*A61L 33/06* (2006.01) *A61L 29/00* (2006.01)
*C08F 293/00* (2006.01) *C08F 297/00* (2006.01)
*A61P 7/02* (2006.01)

(86) International application number:
**PCT/JP2016/057182**

(87) International publication number:
**WO 2016/143787 (15.09.2016 Gazette 2016/37)**

(54) **ANTITHROMBOTIC BLOCK COPOLYMER**
ANTITHROMBOTISCHES BLOCKCOPOLYMER
COPOLYMÈRE BLOC ANTITHROMBOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2015 JP 2015047638**

(43) Date of publication of application:
**17.01.2018 Bulletin 2018/03**

(73) Proprietors:
- **National University Corporation Yamagata University**
  **Yamagata-shi, Yamagata 990-8560 (JP)**
- **Otsuka Chemical Co., Ltd.**
  **Osaka-shi,**
  **Osaka 540-0021 (JP)**

(72) Inventors:
- **TANAKA, Masaru**
  **Yonezawa-shi**
  **Yamagata 992-8510 (JP)**
- **ISHITOBI, Hiroyuki**
  **Tokushima-shi**
  **Tokushima 771-0193 (JP)**

(74) Representative: **Vossius & Partner**
**Patentanwälte Rechtsanwälte mbB**
**Siebertstrasse 3**
**81675 München (DE)**

(56) References cited:
**EP-A1- 1 541 592** **EP-A1- 2 071 584**
**JP-A- S 529 087** **JP-A- 2006 117 713**
**JP-A- 2007 217 516** **JP-A- 2008 194 362**
**JP-A- 2008 280 421** **JP-A- 2008 289 864**
**JP-A- 2016 050 266** **US-A- 4 929 510**
**US-A1- 2008 003 253**

- **KAZUNORI KATAOKA ET AL: "Minimized platelet interaction with poly(2-hydroxyethyl methacrylate-block-4-bis(trimethylsilyl)me thylstyrene) hydrogel showing anomalously high free water content", JOURNAL OF BIOMATERIALS SCIENCE. POLYMER EDITION., vol. 9, no. 2, 1 January 1998 (1998-01-01) , pages 111-129, XP055506642, NL ISSN: 0920-5063, DOI: 10.1163/156856298X00460**
- **ETSUKO HIROTA ET AL: "Study on blood compatibility with poly(2-methoxyethylacrylate)-relationship between surface structure, water structure, and platelet compatibility in 2-methoxyethylacrylate/2-hydroxyethylmetha crylate diblock copolymer", JOURNAL OF BIOMEDICAL MATERIALS RESEARCH. PART A, vol. 76A, no. 3, 1 March 2006 (2006-03-01) , pages 540-550, XP055502609, HOBOKEN, NY, US ISSN: 1549-3296, DOI: 10.1002/jbm.a.30563**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- OKANO T ET AL: "EFFECT OF HYDROPHILIC AND HYDROPHOBIC MICRODOMAINS ON MODE OF INTERACTION BETWEEN BLOCK POLYMER AND BLOOD PLATELETS", JOURNAL OF BIOMEDICAL MATERIALS RESEARCH, WILEY, NEW YORK, NY, US, vol. 15, no. 3, 1 May 1981 (1981-05-01), pages 393-402, XP001015378, ISSN: 0021-9304, DOI: 10.1002/JBM.820150310
- OKANO TERUO ET AL: "Prevention of changes in platelet cytoplasmic free calcium levels by interaction with 2-hydroxyethyl methacrylate/styrene block copolymer surfaces", JOURNAL OF BIOMEDICAL MATERIALS RESEARCH, vol. 27, no. 12, 1993, pages 1519-1525, XP9507997, ISSN: 0021-9304
- RUI ZHANG ET AL: "Reduced Hydrophobic Interaction of Polystyrene Surfaces by Spontaneous Segregation of Block Copolymers with Oligo (Ethylene Glycol) Methyl Ether Methacrylate Blocks: Force Measurements in Water Using Atomic Force Microscope with Hydrophobic Probes", LANGMUIR, vol. 24, no. 10, 1 May 2008 (2008-05-01), pages 5527-5533, XP055506645, US ISSN: 0743-7463, DOI: 10.1021/la703934u
- AKIRA MOCHIZUKI ET AL: "Study of the water structure in poly(methyl methacrylate-block-2-hydroxyethyl methacrylate) and its relationship to platelet adhesion on the copolymer surface", JOURNAL OF BIOMATERIALS SCIENCE. POLYMER EDITION., vol. 26, no. 12, 30 June 2015 (2015-06-30) , pages 750-765, XP055506379, NL ISSN: 0920-5063, DOI: 10.1080/09205063.2015.1056457
- HAN SEOK ET AL: "Synthesis of Thermally Sensitive Water-Soluble Polymethacrylates by Living Anionic Polymerizations of Oligo(ethylene glycol) Methyl Ether Methacrylates", MACROMOLECULES, AMERICAN CHEMICAL SOCIETY, WASHINGTON, DC, UNITED STATES, vol. 36, no. 22, 1 January 2003 (2003-01-01), pages 8312-8319, XP002458836, ISSN: 0024-9297, DOI: 10.1021/MA0347971
- Masaru Tanaka ET AL: "The roles of water molecules at the biointerface of medical polymers", POLYMER JOURNAL, vol. 45, no. 7, 30 January 2013 (2013-01-30), pages 701-710, XP055661848, JP ISSN: 0032-3896, DOI: 10.1038/pj.2012.229

## Description

Technical Field

The present invention relates to an antithrombotic block copolymer composition and a block copolymer for use as an antithrombotic agent.

Background Art

Polymers, ceramics, glasses, metals, and like materials having excellent mechanical properties (high strength, high elasticity or flexibility) and moldability have been used for medical devices. Among these, medical devices used in contact with blood, such as artificial kidney membranes, plasma-separating membranes, artificial lung membranes, catheters, stents, and artificial blood vessels, are required to have antithrombogenicity.

However, most of the above materials do not have antithrombogenicity. When these materials are used, they must be used with an anticoagulant agent, such as heparin.

However, heparin is known to have adverse effects on blood, such as a reduction in the number of platelets. Therefore, there is a time constraint on the continuous usage of heparin. Further, since a raw material for producing heparin is generally obtained from bovine or porcine intestinal mucosa, infection risk has been cited.

To solve such problems, a method comprising coating the surface in contact with blood with a heparin-containing material and immobilizing the heparin has been used to achieve antithrombogenicity while making use of the characteristics of various materials. However, even this method has a problematic reduction in antithrombogenicity due to the elution of heparin. Thus, there is a need to develop a heparin-free coating agent having excellent antithrombogenicity.

On the other hand, biocompatible polymers having antithrombogenicity are known to have water, called intermediate water, which is weakly bound to polymer chains by interactions with the polymer chains. The antithrombogenicity due to the presence of this intermediate water is presumed to be achieved based on the following mechanism. Biological components (e.g., blood cells, platelets, plasma, and like blood-derived proteins) form a hydration shell and are thereby stabilized in blood. However, when unfreezable water on a material surface is contacted with the hydration shell and disrupts the hydration shell, adsorption of biological components on the material surface occurs. The presence of the intermediate water between the hydration shell of a biological component and unfreezable water on the material surface prevents direct contact between the hydration shell and unfreezable water, thereby exhibiting antithrombogenicity.

For example, a homopolymer of 2-methacryloyloxyethyl phosphorylcholine (hereinafter referred to as "MPC") and a homopolymer of 2-methoxyethyl acrylate (hereinafter referred to as "MEA") are known as biocompatible polymers having antithrombogenicity.

However, because the homopolymer of MPC is soluble in water and readily dissolves upon contact with a body fluid, such as blood, the homopolymer of MPC cannot be used as a medical material. On the other hand, the homopolymer of MEA is in a rubber state at room temperature, and thus has poor morphological stability.

Accordingly, Patent Literature (PTL) 1 proposes a medical device comprising a base material whose surface is coated with a random copolymer of MPC and a hydrophobic monomer.

Patent Literature (PTL) 2 proposes a medical polymer material comprising a base material whose surface is coated with a material obtained by crosslinking a random copolymer of MPC and an epoxy-containing monomer with a compound having two or more amino groups and/or two or more carboxyl groups.

Patent Literature (PTL) 3 proposes a medical instrument comprising a base material whose surface is coated with a block copolymer comprising MEA and a (meth)acrylamide or a derivative thereof.

The technique disclosed in PTL 1 incurs few problems if the contact time with blood is short. However, since the adhesion of the coating layer (coating) of a random copolymer to the base material is weak, long-term use may result in gradual elution of the copolymer coating into blood or omission of the copolymer from the base material surface, thus leading to a problem such that blood compatibility does not last long.

The technique disclosed in PTL 2, in which a copolymer is immobilized by a covalent bond, requires a pretreatment on the base material surface and involves many steps, and is therefore complicated.

The technique disclosed in PTL 3 has insufficient adhesion to the base material because of the use of N,N-substituted (meth)acrylamide, which is more hydrophilic than MEA. Therefore, long-term excellent antithrombogenicity is difficult to obtain.

PTL 4 discloses a polymer electrolyte which comprises an ionic liquid (A) and a block copolymer (B) as essential ingredients, which block copolymer (B) comprises one or more of polymer block (s) (P) being compatible with ionic liquid (A) and one or more of polymer block(s) (Q) being incompatible with ionic liquid (A).

PTL 5 refers to a polymer block represented by the general formula (1), a binary block copolymer including a polymer block represented by the general formula (2), and [B] a polystyrene-based polymer.

NPTL 1 describes the reduced hydrophobic interaction of polystyrene surfaces by spontaneous segregation

of block copolymers with oligo (ethylene glycol) methyl ether methacrylate blocks: force measurements in water using atomic force microscope with hydrophobic probes.

The roles of water molecules at the biointerface of medical polymers are discussed in NPTL 2.

Thus, there is a need to develop a block copolymer that is satisfactory in terms of both adhesion to a base material and antithrombogenicity.

Citation List

PTL

PTL 1: JPH3-039309A
PTL 2: JPH7-184989A
PTL 3: JP2013-056146A
PTL 4: EP 2 071 584 A
PTL 5: JP2006-117713A

NPTL

NPTL 1: Langmuir, 24 (10), 2008, 5527-5533
NPTL 2: Polymer Journal, 45(7), 2013, 701-710

Summary of Invention

Technical Problem

An object of the present invention is to provide a block copolymer composition wherein the block copolymer has excellent adhesion to a base material, and imparts excellent antithrombogenicity to the base material surface. Another object of the present invention is to provide a medical device comprising the block copolymer.

Solution to Problem

The present inventors conducted extensive research to achieve the above object. As a result, the inventors found that excellent antithrombogenicity can be imparted to the surface of a base material, and that enhanced adhesion of a block copolymer to the base material can also be achieved by using a block copolymer comprising a specific structural unit capable of containing intermediate water. The present inventors have accomplished the present invention through further research based on the above findings.

The invention is defined by the appended claims. The block copolymer according to the present invention can be prepared using a living radical polymerization, the method comprising:

polymerizing either A block-forming monomer(s) or B block-forming monomer(s) to form a first block polymer; and thereafter polymerizing the other of the A block-forming monomer(s) or B block-forming monomer(s) to form a second block polymer.

An organic tellurium compound is preferably used in the living radical polymerization.

Advantageous Effects of Invention

According to the present invention, a block copolymer composition wherein the block copolymer has excellent adhesion to a base material and imparts excellent antithrombogenicity to a base material surface can be provided. Further, a medical device that has excellent adhesion to a base material and excellent antithrombogenicity can be provided by using the block copolymer.

Brief Description of Drawings

Fig. 1 shows the peaks in the differential scanning calorimetry (DSC) profile of the block copolymer obtained in Example 1.

Fig. 2 shows the peaks in the differential scanning calorimetry (DSC) profile of the block copolymer obtained in Comparative Example 4.

## Description of Embodiments

A preferred embodiment of the present invention is described below. The following descriptions of the embodiment are given by way of illustration.

### 1. Block Copolymer

The block copolymer useful in the present invention comprises an A block and a B block. The A block is capable of containing intermediate water. The B block is more hydrophobic than the A block.
The A block can also be paraphrased as "A segment." The B block can also be paraphrased as "B segment."
Each constituent etc. of the block copolymer of the present invention are explained below.

### 1-1. A Block

The A block is a polymer block comprising a vinyl monomer-derived structural unit capable of containing intermediate water, as defined in the claims.
The "vinyl monomer-derived structural unit capable of containing intermediate water" refers to a structural unit that is formed by radical polymerization of a vinyl monomer, and that can contain intermediate water. Specifically, the "vinyl monomer-derived structural unit" refers to a structural unit obtained by conversion of a radically polymerizable carbon-carbon double bond in a vinyl monomer to a carbon-carbon single bond.
In the present invention, intermediate water refers to water in a state in which an exothermic peak based on a low-temperature crystallization of water is observed at or below 0°C in an elevated temperature process from -100°C in measurement using a differential scanning calorimeter (DSC). The differential scanning calorimeter is not particularly limited. For example, a DSC-7000 instrument (produced by Seiko Instruments Inc.) can be used.
The calorific value of the exothermic peak is not particularly limited. For example, in view of the presence of a sufficient amount of intermediate water, the calorific value is preferably 1 J/g or more, more preferably 1 to 120 J/g, and still more preferably 3 to 100 J/g.
The vinyl monomer used as a starting material for the A block is selected so that a polymer block comprising a vinyl monomer-derived structural unit capable of containing intermediate water can be obtained. Examples include vinyl monomers represented by the following formulas (1) to (7) whereby only formulas (1) and (3) are according to the invention. Formulas (2), (4), as well as (5) to (7) are given for reference purposes:

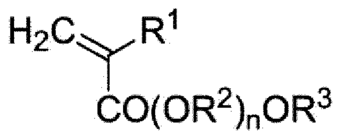 (1)

(wherein $R^1$ is a hydrogen atom (invention) or methyl (reference), $R^2$ is alkylene having 2 or 3 carbon atoms, $R^3$ is methyl or ethyl,
n is an integer of 1 to 10;according to the invention n is 1; n is 2 to 10 are given for reference);

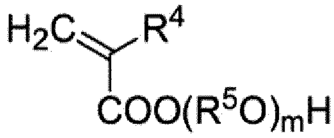 (2)

($R^4$ is a hydrogen atom or methyl,
$R^5$ is alkylene having 2 or 3 carbon atoms, and
m is an integer of 2 to 13);

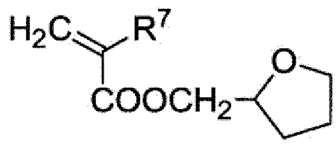

(3)

(wherein $R^7$ is a hydrogen atom or methyl);

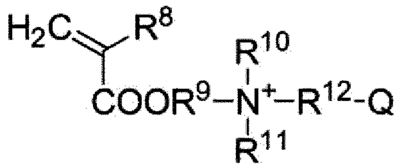

(4)

($R^8$ is a hydrogen atom or methyl,
$R^9$ and $R^{12}$ are independently alkylene having 1 to 6 carbon atoms, $R^{10}$ and $R^{11}$ are independently alkyl having 1 to 4 carbon atoms, and Q is carboxylate-anion ($COO^-$) or sulfo-anion ($SO_3^-$)) ;

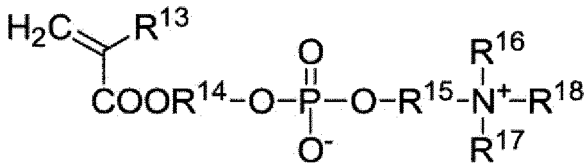

(5)

($R^{13}$ is a hydrogen atom or methyl,
$R^{14}$ and $R^{15}$ are independently alkylene having 1 to 6 carbon atoms, and
$R^{16}$, $R^{17}$, and $R^{18}$ are independently alkyl having 1 to 4 carbon atoms);

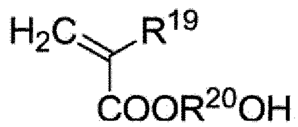

(6)

(wherein $R^{19}$ is a hydrogen atom or methyl, and
$R^{20}$ is alkylene having 2 or 3 carbon atoms); and

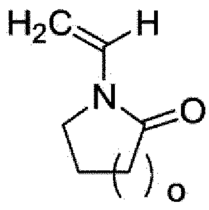

(7)

(wherein o is an integer of 1 to 3).

The block copolymers produced by using vinyl monomers represented by formulas (1) to (7) contain, as the A block, structural units represented by the following formulas (1') to (7'):

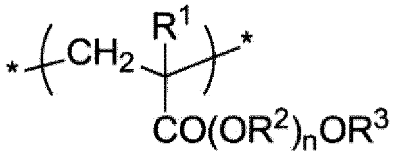

(1')

(wherein $R^1$, $R^2$, $R^3$, and n are as defined above, and each * represents a bond);

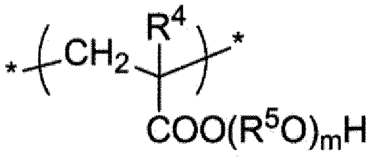

(2')

(wherein $R^4$, $R^5$, and m are as defined above, and each * represents a bond);

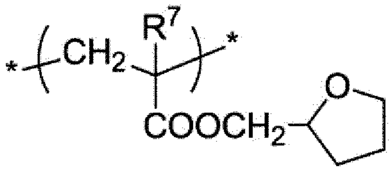

(3')

(wherein $R^7$ is as defined above, and each * represents a bond);

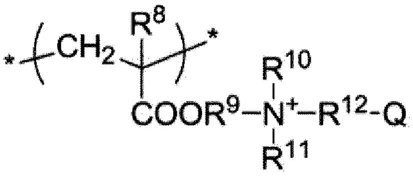

(4')

(wherein $R^8$, $R^9$, $R^{10}$, $R^{11}$, $R^{12}$, and Q are as defined above, and each * represents a bond);

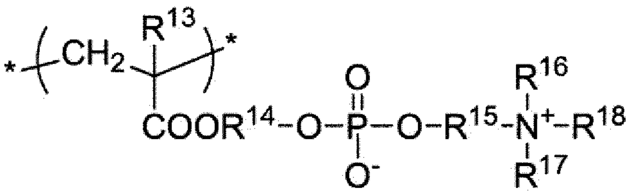

(5')

(wherein $R^{13}$, $R^{14}$, $R^{15}$, $R^{16}$, $R^{17}$, and $R^{18}$ are as defined above, and each * represents a bond);

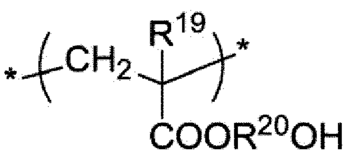

(6')

(wherein $R^{19}$ and $R^{20}$ are as defined above, and each * represents a bond); and

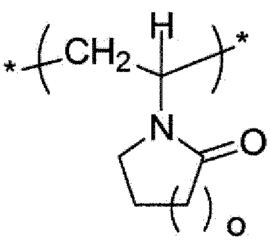

(7')

(wherein o is as defined above, and each * represents a bond).

Examples of compounds represented by Formula (1) include a wide variety of known compounds that fall within the scope of compounds of Formula (1). Among these compounds, 2-methoxyethyl acrylate, 2-ethoxyethyl acrylate, 2-(2-ethoxyethoxy)ethyl acrylate, polyethylene glycol monomethyl acrylate, polypropylene glycol monomethyl acrylate, polyethylene glycol monomethyl methacrylate, and polypropylene glycol monomethyl methacrylate are preferable.
Examples of compounds represented by Formula (2) include a wide variety of known compounds that fall within the scope of compounds of Formula (2). Among these compounds, polyethylene glycol monoacrylate, polypropylene glycol monoacrylate, polyethylene glycol monomethacrylate, and polypropylene glycol monomethacrylate are preferable.
Examples of compounds represented by Formula (3) include a wide variety of known compounds that fall within the scope of compounds of Formula (3). Among these compounds, tetrahydrofurfuryl acrylate is preferable.
Examples of compounds represented by Formula (4) include a wide variety of known compounds that fall within the scope of compounds of Formula (4). Among these compounds, N-methacryloiloxyethyl-N,N-dimethylammonium-α-N-methylcarboxybetaine and N-(3-sulfopropyl)-N-(meth)acryloyloxyethyl-N,N-dimethylammonium betaine are preferable.
Examples of compounds represented by Formula (5) include a wide variety of known compounds that fall within the scope of compounds of Formula (5). Among these, 2-methacryloiloxyethyl phosphorylcholine is preferable.
Examples of compounds represented by Formula (6) include a wide variety of known compounds that fall within the scope of compounds of Formula (6). Among these, 2-hydroxyethyl methacrylate is preferable.
Examples of compounds represented by Formula (7) include a wide variety of known compounds that fall within the scope of Formula (7). Among these, 1-vinyl-2-pyrrolidone is preferable.
The vinyl monomers used to prepare the A block may be used singly, or in a combination of two or more. For example, the A block may comprise a copolymer of a structural unit comprising an a1 block and a structural unit comprising an a2 block.
The A block may consist of only a vinyl monomer-derived structural unit as described above, or may further comprise one or more other structural units, as long as the A block has intermediate water when the obtained block copolymer contains water. When the A block contains other structural units, the amount of the other structural units in the A block is preferably 20 mol% or less. Each structural unit of the A block may be contained in any form, such as a random copolymer or a block copolymer. In terms of uniformity, each structural unit is preferably contained in the form of a random copolymer.

1-2. B Block

The B block is a polymer block that is more hydrophobic than the A block, and that comprises a vinyl monomer-derived structural unit as defined in the claims.
The vinyl monomer can be selected from known vinyl monomers.
The term "hydrophobic" refers to a property of exhibiting a weak interaction with water, and having low affinity with water. Accordingly, the ratio miscible with water, i.e., solubility, serves as a hydrophobic index.
The vinyl monomer for obtaining the B block that is more hydrophobic than the A block usually has a solubility in 20°C water of less than 50 mass%, preferably less than 40 mass%, and more preferably less than 30 mass%.
According to the invention, a vinyl monomer used in the B block is at least one selected from (meth) acrylic acid alkyl esters, alicyclic alkyl esters of (meth)acrylic acid, (meth)acrylic acid aryl esters, N,N-disubstituted aminoalkyl (meth)acrylate;and aromatic vinyl monomers. Heterocyclic heterocyclic ring-containing unsaturated monomers are given for reference.
In the present invention, "(meth)acrylic" means "at least one of acrylic and methacrylic." For example, "(meth)acrylic acid" means "at least one of acrylic acid and methacrylic acid."
In this specification, "n-" means normal, "s-" means secondary (sec-), and "t-" means tertiary (tert-).
The (meth)acrylic acid alkyl ester is not particularly limited. Examples include (meth)acrylic acid alkyl esters, such as methyl (meth)acrylate, ethyl (meth)acrylate, n-propyl (meth)acrylate, isopropyl (meth)acrylate, n-butyl (meth)acrylate, isobutyl (meth)acrylate, s-butyl (meth)acrylate, t-butyl (meth)acrylate, n-hexyl (meth)acrylate, n-octyl (meth)acrylate, isooctyl (meth)acrylate, 2-ethylhexyl (meth)acrylate, n-nonyl (meth)acrylate, isononyl (meth)acrylate, n-decyl (meth)acrylate, isodecyl (meth)acrylate, n-dodecyl (meth)acrylate, n-stearyl (meth)acrylate.
The alicyclic alkyl ester of (meth)acrylic acid is not particularly limited. Examples include cyclohexyl (meth)acrylate, cyclohexylmethyl (meth)acrylate, cyclododecyl (meth)acrylate, bornyl (meth)acrylate, isobornyl (meth)acrylate, dicyclopentanyl (meth)acrylate, dicyclopentenyl (meth)acrylate, dicyclopentenyloxyethyl (meth)acrylate.
The (meth)acrylic acid aryl ester is not particularly limited. Examples include benzyl (meth)acrylate, phenyl (meth) acrylate, phenoxyethyl (meth)acrylate.
Examples of N,N-disubstituted aminoalkyl (meth)acrylates include N,N-dimethylaminopropyl (meth)acrylate,

N,N-dimethylaminobutyl (meth)acrylate, N,N-diethylaminoethyl (meth)acrylate, N,N-diethylaminopropyl (meth)acrylate, N,N-diethylaminobutyl (meth)acrylate, N,N-dipropylaminoethyl (meth)acrylate, N,N-dibutylaminoethyl (meth)acrylate, N,N-dibutylaminopropyl (meth)acrylate, N,N-dibutylaminobutyl (meth)acrylate, N,N-dihexylaminoethyl (meth)acrylate, N,N-dioctylaminoethyl (meth)acrylate.

The aromatic vinyl monomer is not particularly limited. Examples include styrene, α-methylstyrene, 2-methylstyrene, 3-methylstyrene, 4-methylstyrene, 2-methoxystyrene, 3-methoxystyrene, 4-methoxystyrene, 2-hydroxymethylstyrene, 1-vinylnaphthalene.

The heterocyclic ring-containing unsaturated monomer is not particularly limited. Examples include 2-vinylpyridine, 3-vinylpyridine, 4-vinylpyridine, and the like.

The vinyl monomers used to prepare the B block may be used singly, or in a combination of two or more. For example, the B block may comprise a copolymer of a structural unit comprising a b1 block and a structural unit comprising a b2 block.

The B block may consist of only a vinyl monomer-derived structural unit as described above, or may further comprise one or more other structural units, as long as the B block is more hydrophobic than the A block. When the B block comprises other structural units, the amount of the other structural units in the B block is preferably 5 mol% or less. Each structural unit of the B block may be contained in any form, such as a random copolymer or a block copolymer. In terms of uniformity, each structural unit is preferably contained in the form of a random copolymer.

## 1-3. Block Copolymer

The block copolymer comprises an A block capable of containing intermediate water, and a B block that is more hydrophobic than the A block, and has intermediate water when the block copolymer contains water. The block copolymer is considered to phase-separate on the surface of a base material in such a manner that the B block is in contact with the base material, and the A block is positioned on the contact surface with the blood and has intermediate water. As a result, excellent antithrombogenicity can be exhibited over a long period of time.

In the block copolymer of the present invention, the molar ratio (A:B) of the A block to the B block is 85:15 to 40:60.

The weight average molecular weight (Mw) of the block copolymer is not particularly limited, and is usually in the range of 10,000 or more, preferably 10,000 to 1,000,000, more preferably 20,000 to 500,000, and still more preferably 30,000 to 100,000.

The molecular weight distribution (PDI) of the block copolymer is preferably 3.0 or less, and more preferably 2.0 or less. In the present invention, the molecular weight distribution (PDI) is determined by the following formula:

(Weight average molecular weight of block copolymer (Mw))/
(Number average molecular weight of block copolymer (Mn)).

A smaller PDI indicates a copolymer with a narrow molecular weight distribution and a more uniform molecular weight. When the value is 1, the molecular weight distribution is the narrowest. Conversely, a larger PDI indicates the presence of many copolymers with a molecular weight smaller than that of the intended copolymer, and elution of low-molecular-weight copolymers is feared.

The arrangement of polymer (A) and polymer (B) in the block copolymer is not particularly limited, as long as the block copolymer has the various properties described above. For example, a diblock type copolymer (A-B), triblock type copolymers (A-B-A and B-A-B), and like block copolymers are preferably used. When an A-B-A type triblock copolymer is formed, the two A blocks at both ends may be the same or different. When a B-A-B type triblock copolymer is formed, the two B blocks at both ends may be the same or different.

The water content of the block copolymer is usually 1 to 50 mass%, preferably 2 to 40 mass%, and more preferably 3 to 35 mass%.

When a base material is coated with the block copolymer to obtain a medical device, the elution rate is usually in the range of 0 to 2 mass%, and preferably 0 to 1 mass%.

## 2. Method for Producing the Block Copolymer

The method for producing the block copolymer is not particularly limited. Examples include block polymerization methods, such as living radical polymerization methods. The block copolymer is obtained by sequentially subjecting monomers to a polymerization reaction by such a block polymerization method.

The method for producing the block copolymer may comprise first producing an A block (an A segment) by a polymerization reaction of monomer(s), and then polymerizing B block (B segment) monomer(s) onto the A block; first producing a B block and then polymerizing A block

monomer(s) onto the B block; or separately producing an A block and a B block by polymerization reactions of monomers, and then coupling the A block and the B block. For example, the method for producing the block copolymer is a method using a living radical polymerization, and comprises the steps of polymerizing either A block-forming monomer(s) or B block-forming monomer(s) to form a first block polymer, and thereafter polymerizing the other of the A block-forming monomer(s) or B block-forming monomer(s) to form a second block polymer.

The arrangement of the A block and B block in the block copolymer is not necessarily limited as long as the resulting block copolymer has the various properties described above. For example, a diblock type polymer (A-B), triblock type polymers (A-B-A and B-A-B), or like block polymers are preferably used.

When conventional radical polymerization methods are used, not only an initiation reaction and a growth reaction, but also a termination reaction and a chain transfer reaction occur. The termination reaction or the chain transfer reaction causes deactivation of the growth terminal, and tends to form a mixture of polymers having various molecular weights and a non-uniform composition. The living radical polymerization method is preferable because this method allows, while maintaining the convenience and general versatility of radical polymerization, the growth terminal to grow without deactivation, due to decreased likelihood of a termination reaction and a chain transfer reaction; accordingly, precise control of the molecular structure and production of a polymer having a uniform composition are easy. The living radical polymerization method includes the following methods that are different in terms of the method for stabilizing the polymerization growth terminal: a method using a transition metal catalyst (the atom transfer radical polymerization (ATRP) method); a method using a sulfur-based reversible chain transfer agent (the reversible addition-fragmentation chain transfer (RAFT) method); a method using an organic tellurium compound (the organotellurium-mediated living radical polymerization (TERP) method); and like methods. Among these methods, the TERP method is preferable in view of monomer diversity, molecular weight control in the polymer range, uniform composition, and coloring.

The TERP method is a method for polymerizing a radical polymerizable compound using an organic tellurium compound as a polymerization initiator. Examples include the methods disclosed in WO2004/14848 and WO2004/14962.

Specific examples include polymerization methods using the following (a) to (d).

(a) an organic tellurium compound represented by Formula (8) shown below,
(b) a mixture of an organic tellurium compound represented by Formula (8) and an azo polymerization initiator,
(c) a mixture of an organic tellurium compound represented by Formula (8) and an organic ditellurium compound represented by Formula (9) shown below, or
(d) a mixture of an organic tellurium compound represented by Formula (8), an azo polymerization initiator, and an organic ditellurium compound represented by Formula (9),

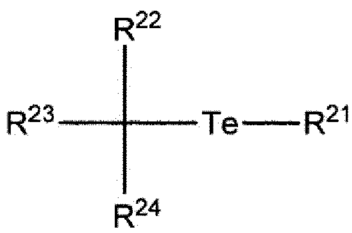

(8)

(wherein $R^{21}$ is alkyl having 1 to 8 carbon atoms, aryl, or heteroaryl, $R^{22}$ and $R^{23}$ are each a hydrogen atom or alkyl having 1 to 8 carbon atoms, $R^{24}$ is alkyl having 1 to 8 carbon atoms, aryl, heteroaryl, acyl, amide, oxycarbonyl, or cyano),

$(R^{21}Te)_2$ (9)

(wherein $R^{21}$ is as defined above) .

Specific examples of organic tellurium compounds represented by Formula (8) include (methyltellanylmethyl)benzene, (methyltellanylmethyl)naphthalene, ethyl-2-methyl-2-methyltellanyl-propionate, ethyl-2-methyl-2-n-butyltellanyl-propionate, (2-trimethylsiloxyethyl)-2-methyl-2-methyltellanyl-propionate, (2-hydroxyethyl)-2-methyl-2-methyltellanyl-propionate, and (3-trimethylsilylpropargyl)-2-methyl-2-methyltellanyl-propionate, and the like.

Specific examples of organic ditellurium compounds represented by Formula (9) include dimethyl ditelluride, diethyl ditelluride, di-n-propyl ditelluride, diisopropyl ditelluride, dichloropropyl ditelluride, di-n-butyl ditelluride, di-s-butyl ditelluride, di-t-butyl ditelluride, dichlorobutyl ditelluride, diphenyl ditelluride, bis-(p-methoxypheny) ditelluride, bis-(p-aminophenyl) ditelluride, bis-(p-nitrophenyl) ditelluride, bis-(p-cyanophenyl) ditelluride, bis-(p-sulfonylphenyl) ditelluride, dinaphthyl ditelluride, dipyridyl ditelluride, and the like.

Any azo polymerization initiators that are used in usual radical polymerization can be used as the azo polymerization initiator. Example include 2,2'-azobis(isobutyronitrile) (AIBN), 2,2'-azobis(2-methylbutyronitrile) (AMBN), 2,2'-azobis(2,4-dimethylvaleronitrile) (ADVN), 1,1'-azobis(1-cyclohexanecarbonitrile) (ACHN), dimethyl-2,2'-azobisisobutyrate (MAIB), 4,4'-azobis(4-cyanovaleric acid) (ACVA), 1,1'-azobis(1-acetoxy-1-phenylethane), 2,2'-azobis(2-methylbutylamide), 2,2'-azobis(4-methoxy-2,4-dimethylvaleronitrile) (V-70), 2,2'-azobis(2-methylamidinopropane) dihydrochloride, 2,2'-azobis[2-(2-imidazolin-2-yl)propane], 2,2'-azobis[2-methyl-N-(2-hydroxyethyl)propionamide], 2,2'-azobis(2,4,4-trimethylpentane), 2-cyano-2-propylazoformamide, 2,2'-azobis(N-butyl-2-methylpropionamide), 2,2'-azobis(N-cyclohexyl-2-methylpropionamide), and the like.

The amount of the compound of Formula (8) in each of (a), (b), (c), and (d) described above can be appropriately adjusted according to the physical properties of the desired block copolymer. The compound of Formula (8) can be typically used in an amount of 0.05 to 50 mmol per mole of the starting monomer.

When the compound of Formula (8) is used with an azo polymerization initiator as described in (b), the amount of the azo polymerization initiator can be typically 0.01 to 10 moles per mole of the compound of Formula (8).

When the compound of Formula (8) is used with the compound of Formula (9) as described in (c), the amount of the compound of Formula (9) can be typically 0.01 to 100 moles per mole of the compound of Formula (8).

When the compound of Formula (8) is used with the compound of Formula (9) and an azo polymerization initiator as described in (d), the amount of the azo polymerization initiator can be typically 0.01 to 100 moles per mole of the total amount of the compound of Formula (8) and the compound of Formula (9).

The polymerization reaction in the production method is performed using a solvent commonly used in radical polymerization (an organic solvent or an aqueous solvent) and stirring the mixture, although the polymerization reaction can also be performed in the absence of a solvent.

Usable organic solvents are not particularly limited. Examples include benzene, toluene, N,N-dimethylformamide (DMF), dimethyl sulfoxide (DMSO), acetone, 2-butanone (methyl ethyl ketone), dioxane, hexafluoroisopropanol, chloroform, carbon tetrachloride, tetrahydrofuran (THF), ethyl acetate, trifluoromethylbenzene, and the like.

Examples of aqueous solvents include water, methanol, ethanol, isopropanol, n-butanol, ethyl cellosolve, butyl cellosolve, 1-methoxy-2-propanol, diacetone alcohol, and the like.

The amount of the solvent can be appropriately adjusted. For example, the amount of the solvent is typically in the range of 0.01 to 50 ml, preferably 0.05 to 10 ml, and more preferably 0.1 to 1 ml, per gram of the starting monomer.

The reaction temperature and the reaction time can be appropriately adjusted according to the molecular weight or molecular weight distribution of the obtained copolymer. The mixture is typically stirred at 0 to 150°C for 1 minute to 100 hours. The TERP method can achieve a high yield and precise molecular weight distribution even at a low polymerization temperature and in a short polymerization time period.

After completion of the polymerization reaction, the desired copolymer can be isolated from the obtained reaction mixture by usual separation and purification means.

## 3. Block Copolymer Composition

The block copolymer composition of the present invention is a composition comprising intermediate water and a block copolymer as defined in the claims.

Water is present inside or on the surface of the block copolymer in the block copolymer composition of the present invention. The following three forms of water are considered to be present:

(1) free water that has a weak interaction with the A block polymer capable of containing intermediate water, and melts at 0°C;
(2) unfreezable water that has a strong interaction with the A block polymer capable of containing intermediate water, and that does not freeze even at -100°C; and
(3) intermediate water that has an intermediate level of interaction, i.e., an interaction stronger than the interaction of free water and weaker than the interaction of unfreezable water, and that freezes at a temperature of less than 0°C.

Biological components, such as hemocytes or like cell proteins, form a hydration shell and are thereby stabilized in blood.

However, conventional polymer materials, which have only an unfreezable water layer on the material surface, are known to destroy the hydration shell of cell proteins, such as hemocytes, thus resulting in adsorption of biological components on the material surface as a thrombus (JP2004-161954A).

In contrast, the block copolymer has intermediate water inside or on the surface of the copolymer. Due to the presence of the intermediate water, the block copolymer is considered to exhibit antithrombogenicity without destroying the hydration shell of cell proteins, such as hemocytes.

Unfreezable water may be present between the block copolymer and intermediate water. Intermediate water

is considered to be present between unfreezable water and the hydration shell of a biological component.

The ratio (mass ratio) of intermediate water and the block copolymer in the block copolymer composition of the present invention is not particularly limited. The ratio of intermediate water to the block copolymer is usually in the range of 1:99 to 30:70, preferably 1.5:98.5 to 25:75, and more preferably 2:98 to 20:80.

The method of producing the block copolymer composition of the present invention can form the block copolymer composition by bringing the block copolymer into contact with a water-containing solution (e.g., water or a biological component such as blood) to allow the block copolymer to contain water upon which water molecules are hydrogen-bonded to polar groups of polymer side chains.

## 4. Antithrombotic Coating Agent

The block copolymer can be used as an antithrombotic coating agent. The antithrombotic coating agent is characterized by containing the block copolymer as defined above. The antithrombotic coating agent can be mixed, dispersed, or dissolved in a solvent, if necessary, and then used.

Examples of usable solvents include water, organic solvents, or mixed solvents thereof. The type and concentration of the solvent used vary depending on, for example, the composition and molecular weight of the obtained block copolymer, and the type, and surface texture of the base material to be coated, and can be suitably selected.

In the present invention, as a base material (for a medical device) subjected to antithrombotic coating, various materials can be used. Examples include polyvinyl chloride, polycarbonate, polyethylene terephthalate, polyethylene, polypropylene, polymethylpentene, thermoplastic polyurethane, thermosetting polyurethane, silicone rubber such as polydimethylsiloxane having a crosslinked portion, polymethylmethacrylate, polyvinylidene fluoride, tetrafluoropolyethylene, polysulfone, polyethersulfone, polyacetal, polystyrene, ABS, and like resins and mixtures of these resins; metals such as stainless steel, titanium, and aluminum; glass; ceramics; and the like.

The base material may be in any shape or form, such as plates, sheets, straw, pipes, fabrics, globular shape, nonwoven fabrics, and porous forms.

The coating agent can form a uniform coating film on the base material.

The method for coating the base material of a medical device etc. with the coating agent is not particularly limited.

Specific examples of coating methods include an immersion method comprising immersing a base material in the composition for a long period of time; a spraying method comprising spraying over a base material; a method of application using a brush, a flocked brush, etc.; a method comprising bringing a base material into contact with a solution of the coating agent; and like methods. Among these methods, a method comprising bringing a base material into contact with a solution of the coating agent is preferable in view of ease of control of a coating layer etc. To coat a base material with the coating agent, one type of such coating methods may be performed several times, or two or more types of such coating methods may be performed several times.

## 5. Medical Device

The medical device is obtained by coating a base material with the antithrombotic coating agent of the present invention. The medical device can be provided with greatly enhanced antithrombogenicity, and the entire medical device surface or a selected portion thereof is coated with the antithrombotic coating agent. The medical device can be used as is after being coated or after being further subjected to a priming treatment with physiological saline or the like.

The medical device is not particularly limited, as long as it is used in direct contact with the blood. Examples include catheters (e.g., catheters, balloons of balloon catheters, and guide wires), artificial blood vessels, blood vessel bypass tubes, artificial valves, blood filters, plasma separation devices, artificial internal organs (e.g., artificial lungs, artificial livers, and artificial hearts), blood transfusion tools, extracorporeal circulation blood circuits, blood bags, adhesion preventing films, wound dressings, and the like.

## Examples

The present invention is described below in detail with reference to Examples. However, the present invention is not limited to the Examples. In the Examples and Comparative Examples below, various physical properties were measured using the following instruments.

### Monomer-to-Polymer Conversion

$^1$H-NMR was measured using a 500-MHz NMR instrument (AVANCE500, produced by Bruker BioSpin). The

monomer-to-polymer conversion was calculated from the integration ratio of vinyl groups of the starting monomers to polymer peaks.

Composition Ratio

$^1$H-NMR was measured using a 500-MHz NMR instrument (AVANCE500, produced by Bruker BioSpin). The composition ratio was calculated from the integration ratio of each polymer peak.

Weight Average Molecular Weight (Mw) and Molecular Weight Distribution (PDI)

A calibration curve was prepared using a GPC system (HLC-8320GPC, produced by Tosoh Corporation), two TSKgel SuperMultipore HZ-H columns ($\phi$4.6 $\times$ 150, produced by Tosoh Corporation, Tokyo, Japan), tetrahydrofuran as a mobile phase, and polystyrene (Tosoh-TSK Standard) as a standard substance to determine the weight average molecular weight (Mw) and the number average molecular weight (Mn) of each sample. The molecular weight distribution (PDI) was calculated from these measurement values.

Example 1: Poly(2-methoxyethyl acrylate)-polystyrene-poly(2-methoxyethyl acrylate)

In a nitrogen-purged glove box, a mixture of 2.05 g (15.8 mmol) of 2-methoxyethyl acrylate (hereinafter referred to as "MEA"), 22.9 $\mu$l (0.10 mmol) of ethyl-2-methyl-2-n-butyltellanyl-propionate (hereinafter referred to as "BTEE"), 4.9 mg (0.020 mmol) of 1,1'-azobis(1-cyclohexanecarbonitrile) (hereinafter referred to as "ACHN"), and 2.05 g of propylene glycol monomethyl ether acetate (hereinafter referred to as "PMA") was prepared. The resulting mixture was allowed to react at 90°C for 21 hours. The monomer-to-polymer conversion was 100%, Mw was 27,370, and PDI was 1.27.

After 0.90 g (8.64 mmol) of styrene (hereinafter referred to as "St"), 12.2 mg (0.050 mmol) of ACHN, and 0.9 g of PMA were added to the obtained solution, the resulting mixture was allowed to react at 90°C for 46 hours. The monomer-to-polymer conversion was 91%, Mw was 37,320, and PDI was 2.0.

After 2.05 g (15.8 mmol) of MEA, 4.9 mg (0.020 mmol) of ACHN, and 2.05 g of PMA were added to the obtained solution. The resulting mixture was allowed to react at 90°C for 26 hours. The monomer-to-polymer conversion was 99%.

After completion of the reaction, the reaction product was subjected to a reprecipitation treatment with heptane, and then dried to obtain 4.06 g of poly(2-methoxyethyl acrylate)-polystyrene-poly(2-methoxyethyl acrylate). Mw was 52,190, PDI was 1.89, and the composition ratio of poly(2-methoxyethyl acrylate)/polystyrene was 81/19.

Example 2: Poly(2-methoxyethyl acrylate)-polystyrene-poly(2-methoxyethylacrylate)

In a nitrogen-purged glove box, a mixture of 1.35 g (10.4 mmol) of MEA, 22.9 $\mu$l (0.10 mmol) of BTEE, 4.9 mg (0.020 mmol) of ACHN, and 1.35 g of PMA was allowed to react at 90°C for 21 hours. The monomer-to-polymer conversion was 100%, Mw was 18,850, and PDI was 1.22.

After 2.30 g (22.1 mmol) of St, 12.2 mg (0.050 mmol) of ACHN, and 2.30 g of PMA were added to the obtained solution, the resulting mixture was allowed to react at 90°C for 46 hours. The monomer-to-polymer conversion was 89%, Mw was 47,500, and PDI was 1.76.

After 1.35 g (10.4 mmol) of MEA, 4.9 mg (0.020 mmol) of ACHN, and 1.35 g of PMA were added to the obtained solution, the resulting mixture was allowed to react at 90°C for 26 hours. After 3.3 mg (0.020 mmol) of 2,2'-azobis(isobutyronitrile) (hereinafter referred to as "AIBN") was added, the resulting mixture was allowed to react at 60°C for 68 hours. The monomer-to-polymer conversion was 98%.

After the completion of the reaction, the reaction product was subjected to a reprecipitation treatment with heptane, and then dried to obtain 4.53 g of poly(2-methoxyethyl acrylate)-polystyrene-poly(2-methoxyethyl acrylate). Mw was 59,830, PDI was 1.75, and the composition ratio of poly(2-methoxyethyl acrylate)/polystyrene was 49/51.

Example 3: Poly(2-methoxyethyl acrylate)-polystyrene

In a nitrogen-purged glove box, a mixture of 3.90 g (30.0 mmol) of MEA, 22.9 $\mu$l (0.10 mmol) of BTEE, 7.3 mg (0.030 mmol) of ACHN, and 3.90 g of PMA was allowed to react at 90°C for 20 hours. The monomer-to-polymer conversion was 100%, Mw was 51,510, and PDI was 1.39.

After 1.10 g (10.6 mmol) of St, 24.4 mg (0.10 mmol) of ACHN, and 1.10 g of PMA were added to the obtained solution, the resulting mixture was allowed to react at 90°C for 22 hours. The monomer-to-polymer conversion was 77%.

After completion of the reaction, the reaction product was subjected to a reprecipitation treatment with heptane, and then dried to obtain 4.07 g of poly(2-methoxyethyl acrylate)-polystyrene. Mw was 54,990, PDI was 2.0, and the composition ratio of poly(2-methoxyethyl acrylate)/polystyrene was 81/19.

Example 4: Poly(2-methoxyethyl acrylate)-polystyrene

In a nitrogen-purged glove box, a mixture of 2.50 g (19.2 mmol) of MEA, 22.9 μl (0.10 mmol) of BTEE, 4.9 mg (0.020 mmol) of ACHN, and 2.50 g of PMA was allowed to react at 90°C for 21 hours. The monomer-to-polymer conversion was 100%, Mw was 32,720, and PDI was 1.27.

After 2.50 g (24.0 mmol) of St, 12.2 mg (0.05 mmol) of ACHN, and 2.50 g of PMA were added to the obtained solution, the resulting mixture was reacted at 90°C for 22 hours. After 12.2 mg (0.05 mmol) of ACHN was added to the mixed solution, the resulting mixture was allowed to react at 90°C for 24 hours. The monomer-to-polymer conversion was 84%.

After completion of the reaction, the reaction product was subjected to a reprecipitation treatment with heptane, and then dried to obtain 4.07 g of poly(2-methoxyethyl acrylate)-polystyrene. Mw was 57,520, PDI was 1.65, and the composition ratio of poly(2-methoxyethyl acrylate)/polystyrene was 49/51.

Example 5 (reference): Poly[2-(2-ethoxyethoxy)ethyl acrylate]-polystyrene

In a nitrogen-purged glove box, a mixture of 5.32 g (28.3 mmol) of 2-(2-ethoxyethoxy)ethyl acrylate (hereinafter referred to as "EEEA"), 33.1 μl (0.14 mmol) of BTEE, 4.7 mg (0.029 mmol) of AIBN, and 5.0 g of propylene glycol monomethyl ether (hereinafter referred to as "MP") was allowed to react at 60°C for 48 hours. The monomer-to-polymer conversion was 99%, Mw was 28,620, and PDI was 1.78.

After 1.90 g (18.2 mmol) of St, 17.6 mg (0.07 mmol) of ACHN, and 2.30 g of PMA were added to the obtained solution, the resulting mixture was allowed to react at 90°C for 24 hours. After 17.6 mg (0.07 mmol) of ACHN was added, the resulting mixture was allowed to react at 90°C for 56 hours. The monomer-to-polymer conversion was 91%.

After completion of the reaction, the reaction product was subjected to a reprecipitation treatment with heptane, and then dried to obtain 5.62 g of poly[2-(2-ethoxyethoxy)ethyl acrylate]-polystyrene. Mw was 32,990, PDI was 1.84, and the composition ratio of poly[2-(2-ethoxyethoxy)ethyl acrylate]/polystyrene was 65/35.

Example 6 (reference): Polyethylene glycol monomethyl acrylate-polystyrene

In a nitrogen-purged glove box, a mixture of 4.20 g (8.7 mmol) of polyethylene glycol monomethyl acrylate (produced by NOF Corporation, Blemmer AME-400, hereinafter referred to "M9EGA"), 23.8 μl (0.10 mmol) of BTEE, 5.1 mg (0.021 mmol) of ACHN, and 4.0 g of MP was allowed to react at 90°C for 24 hours. The monomer-to-polymer conversion was 96%, Mw was 16,680, and PDI was 1.46.

After 1.50 g (9.6 mmol) of St, 12.7 mg (0.05 mmol) of ACHN, and 2.0 g of PMA were added to the obtained solution, the resulting mixture was allowed to react at 90°C for 29 hours. After 8.5 mg (0.05 mmol) of AIBN was added, the resulting mixture was allowed to react at 60°C for 62 hours. The monomer-to-polymer conversion was 89%.

After completion of the reaction, the reaction product was subjected to a reprecipitation treatment with heptane, and then dried to obtain 4.60 g of polyethylene glycol monomethyl acrylate-polystyrene. Mw was 31,170, PDI was 1.79, and the composition ratio of polyethylene glycol monomethyl acrylate/polystyrene was 40/60.

Example 7: Poly(tetrahydrofurfuryl acrylate)-polystyrene

In a nitrogen-purged glove box, a mixture of 5.51 g (35.3 mmol) of tetrahydrofurfuryl acrylate (hereinafter referred to as "THFA"), 37.9 μl (0.17 mmol) of BTEE, 5.4 mg (0.033 mmol) of AIBN, and 5.10 g of anisole was reacted at 60°C for 64 hours. The monomer-to-polymer conversion was 100%, Mw was 45,500, and PDI was 1.49.

After 2.76 g (26.5 mmol) of St, 12.1 mg (0.05 mmol) of ACHN, and 3.50 g of anisole were added to the obtained solution, the resulting mixture was allowed to react at 90°C for 25 hours. After 12.1 mg (0.05 mmol) of ACHN was added, the resulting mixture was allowed to react at 90°C for 48 hours. The monomer-to-polymer conversion was 88%.

After completion of the reaction, the reaction product was subjected to a reprecipitation treatment with heptane, and then dried to obtain 7.00 g of poly(tetrahydrofurfuryl acrylate)-polystyrene. Mw was 72,540, PDI was 2.76, and the composition ratio of polytetrahydrofurfuryl acrylate/polystyrene was 65/35.

Example 8 (reference): Poly(1-vinyl 2-pyrrolidone)-polystyrene

In a nitrogen-purged glove box, a mixture of 4.70 g (42.3 mmol) of 1-vinyl-2-pyrrolidone (hereinafter referred to as "VP"), 41.2 μl (0.18 mmol) of BTEE, 5.9 mg (0.036 mmol) of AIBN, and 4.50 g of anisole was allowed to react at 60°C for 64 hours. The monomer-to-polymer conversion was 100%, Mw was 30,490, and PDI was 1.36.

After 4.30 g (41.3 mmol) of St, 13.2 mg (0.05 mmol) of ACHN, and 5.50 g of anisole were added to the obtained solution, the resulting mixture was reacted at 90°C for 24 hours. After 13.2 mg (0.05 mmol) of ACHN was added, the

resulting mixture was allowed to react at 90°C for 24 hours. The monomer-to-polymer conversion was 89%.

After completion of the reaction, the reaction product was subjected to a reprecipitation treatment with heptanes and then dried to obtain 8.45 g poly(1-vinyl 2-pyrrolidone)-polystyrene. Mw was 75,340, PDI was 2.38, and the composition ratio of poly(1-vinyl 2-pyrrolidone)/polystyrene was 53/47.

Comparative Example 1: Poly(2-methoxyethyl acrylate)

In a nitrogen-purged glove box, 5.0 g (38.4 mmol) of MEA, a mixture of 14.3 μl (0.063 mmol) of BTEE, 4.6 mg (0.020 mmol) of ACHN, and 5.0 g of PMA was allowed to react at 90°C for 23 hours. The monomer-to-polymer conversion was 100%.

After completion of the reaction, the reaction product was subjected to a reprecipitation treatment with heptane, and then dried to obtain 4.49 g of poly(2-methoxyethyl acrylate).
Mw was 84,800 and PDI was 1.96.

Comparative Example 2: Polystyrene

In a nitrogen-purged glove box, a mixture of 6.0 g (57.6 mmol) of St, 3.9 μl (0.017 mmol) of BTEE, and 2.1 mg (0.009 mmol) of ACHN was allowed to react at 90°C for 22 hours. The monomer-to-polymer conversion was 69%.

After completion of the reaction, the reaction product was subjected to a reprecipitation treatment with heptane, and then dried to obtain 3.52 g of polystyrene. Mw was 176,000 and PDI was 1.57.

Comparative Example 3: Poly(2-methoxyethyl acrylate)-poly(N,N-dimethylacrylamide)-poly(2-methoxyethyl acrylate)

In a nitrogen-purged glove box, a mixture of 0.794 g (6.10 mmol) of MEA, 5.61 μl (0.0245 mmol) of BTEE, 0.8 mg (0.0049 mmol) of AIBN, and 0.80 g of PMA was allowed to react at 60°C for 24 hours. The monomer-to-polymer conversion was 95%.

After 3.63 g (36.59 mmol) of N,N-dimethylacrylamide (DMAAm), 0.8 mg (0.0049 mmol) of AIBN, and 8.0 g of PMA were added to the obtained solution, the resulting mixture was allowed to react at 60°C for 23 hours. The monomer-to-polymer conversion was 100%.

After 0.794 g (6.10 mmol) of MEA, 0.8 mg (0.0049 mmol) of AIBN, and 2.0 g of PMA were added to the obtained solution, the resulting mixture was allowed to react at 60°C for 23 hours. After 0.8 mg (0.0049 mmol) of AIBN was added to this mixed solution, the resulting mixture was allowed to react at 60°C for 16 hours. The monomer-to-polymer conversion was 96%.

After completion of the reaction, the reaction product was subjected to a reprecipitation treatment with heptane, and then dried to obtain 4.71 g of poly(2-methoxyethyl acrylate)-poly(N,N-dimethylacrylamide)-poly(2-methoxyethyl acrylate). The composition ratio of poly(2-methoxyethyl acrylate)/poly(N,N-dimethylacrylamide) was 27/73.

Comparative Example 4: Poly(2-methoxyethyl acrylate-styrene)

In a nitrogen-purged glove box, a mixture of 4.10 g (31.5 mmol) of MEA, 0.90 g (8.64 mmol) of St, 22.9 μl (0.10 mmol) of BTEE, 12.2 mg (0.050 mmol) of ACHN, and 5.0 g of PMA was allowed to react at 90°C for 23 hours. After 4.9 mg (0.02 mmol) of ACHN was added, the resulting mixture was allowed to react at 90°C for 24 hours. The monomer-to-polymer conversion was 100%.

After completion of the reaction, the reaction product was subjected to a reprecipitation treatment with heptane, and then dried to obtain 4.25 g of poly(2-methoxyethyl acrylate-styrene). Mw was 59,530, PDI was 1.41, and the composition ratio of poly(2-methoxyethyl acrylate)/polystyrene was 77/23.

Comparative Example 5: Poly(2-methoxyethyl acrylate-styrene)

In a nitrogen-purged glove box, a mixture of 2.80 g (21.5 mmol) of MEA, 2.20 g (21.1 mmol) of St, 22.9 μl (0.10 mmol) of BTEE, 12.2 mg (0.050 mmol) of ACHN, and 5.0 g of PMA was allowed to react at 90°C for 23 hours. After 4.9 mg (0.02 mmol) of ACHN was added to the mixed solution, the resulting mixture was allowed to react at 90°C for 47 hours. The conversion of St was 100%, and the conversion of MEA was 89%.

After completion of the reaction, the reaction product was subjected to a reprecipitation treatment with heptane, and then dried to obtain 4.0 g of poly(2-methoxyetyl acrylate-styrene). Mw was 61,450, PDI was 1.58, and the composition ratio of poly(2-methoxyethyl acrylate)/polystyrene was 47/53.

Test Example 1: Calorific Value Measurement of Exothermic Peak Based on Low-Temperature Crystallization

The polymers obtained in the Examples and the Comparative Examples were immersed in water so that the polymers contained water. A prescribed amount of each sample after containing water was weighed out and thinly spread over the bottom of an aluminum pan whose weight had been measured beforehand. The sample was cooled from room temperature to -100°C, then maintained for 10 minutes, and heated from -100°C to 50°C at a temperature-rise rate of 5.0°C/min, during which the endothermic and exothermic amounts were measured using a differential scanning calorimeter (DSC-7000, produced by Seiko Instruments Inc.). Table 1 below shows the calorific value of the exothermic peak based on low-temperature crystallization at 0°C or less.

Test Example 2: Measurement of Water Content

The water content of each sample used in the calorific value measurement of the exothermic peak based on low-temperature crystallization was measured in the following manner. A prescribed amount of each sample after containing water was weighed out and thinly spread over the bottom of an aluminium oxide pan whose weight had been weighed beforehand. After DSC measurement, pinholes were formed in the aluminum pan, and each sample was vacuum-dried. The water content was determined by measuring the weight before and after the drying. The water content (WC) of each sample was calculated according to the following formula (I):

$$WC = (W_1 - W_0)/W_1) \times 100$$

(wherein WC is water content (mass%), $W_0$ is dry weight (g) of a sample, and $W_1$ is wet weight (g) of a sample) Table 1 shows the water content of each sample.

Table 1

| | Example 1 | Example 2 | Example 3 | Example 4 |
|---|---|---|---|---|
| Calorific value of the exothermic peak based on low-temperature crystallization (J/g) | 7.23 | 5.62 | 7.64 | 5.92 |
| Water content (mass%) | 11.34 | 9.05 | 10.99 | 6.27 |
| | Example 5 | Example 6 | Example 7 | Example 8 |
| Calorific value of the exothermic peak based on low-temperature crystallization (J/g) | 7.35 | 25.3 | 4.67 | 17.7 |
| Water content (mass%) | 4.20 | 26.97 | 4.20 | 31.12 |
| | Comparative Example 1 | Comparative Example 4 | Comparative Example 5 | |
| Calorific value of the exothermic peak based on low-temperature crystallization (J/g) | 13.4 | - | - | |
| Water content (mass%) | 9.78 | 16.59 | 7.64 | |

Test Example 3: Platelet Adhesion Test

The substrates (base material: polyethylene terephthalate) that were spin-coated with the polymers obtained in the Examples and Comparative Examples were cut into 8 mm × 8 mm squares, and fixed to the specimen stage of a scanning electron microscope (SU8000, produced by Hitachi High-Technologies Corporation).

Human blood was centrifuged at 1500 rpm for 5 minutes, and the supernatant was recovered as platelet-rich plasma (PRP). The rest of the blood was further centrifuged at 4000 rpm for 10 minutes, and the supernatant was

recovered as platelet-poor plasma (PPP). The PPP was diluted 800 times with a phosphate-buffered saline (PBS) solution. While the platelet count was confirmed under a microscope, the PRP was further diluted to prepare a platelet solution with a platelet concentration of $4 \times 10^7$ cells/mL. This platelet solution was added dropwise to each substrate in an amount of 200 μL, and the substrate was allowed to stand at 37°C for 1 hour.

Each substrate was then washed twice with a PBS solution, immersed in a 1% glutaraldehyde solution, and allowed to stand at 37°C for 2 hours to fix platelets. The platelet-fixed sample was washed by immersion in a PBS solution for 10 minutes, then in PBS:water = 1:1 for 8 minutes, thereafter in water for 8 minutes, and again in water for 8 minutes. Each sample was air-dried at room temperature. The number of adhered platelets was counted using SEM.

The number of platelets adhered to polyethylene terephthalate (PET) was defined as 100. Table 2 shows the measurement results.

Table 2

| | Example 2 | Comparative Example 1 | Comparative Example 2 | PET |
|---|---|---|---|---|
| Platelet count | 22.41 | 4.95 | 134.29 | 100 |

Test Example 4: Adhesion Test (Coating Evaluation)

Test Example 4-1. Base Material: Soda Glass

5 cm × 5 cm soda glass (0.7 mm in thickness) was spin-coated with each of the dilute solutions of the polymers obtained in the Examples and the Comparative Examples, and then dried in a heated-air oven at 90°C for 30 minutes to obtain a coated base material. The obtained coated base material was observed with the naked eye, and under a light microscope and an atomic force microscope. When the surface has a uniform coating, it was evaluated as A. When floating of the coating layer on the coated base material (i.e., poor adhesion) was observed, it was evaluated as B. Table 3 shows the results.

Table 3

| | Example 2 | Comparative Example 1 | Comparative Example 3 | Comparative Example 5 |
|---|---|---|---|---|
| Base material: soda glass | A | B | B | B |

Test Example 4-2. Base material: Polypropylene (PP)

Evaluation was performed in the same manner as in Test Example 4-1, except that polypropylene (PP) resin plates were used as the base material. Table 4 shows the results.

Table 4

| | Example 2 | Example 4 | Example 5 | Example 6 | Example 7 |
|---|---|---|---|---|---|
| Base material: PP | A | A | A | A | A |
| | Example 8 | Comparative Example 1 | Comparative Example 3 | Comparative Example 5 | |
| Base material: PP | A | B | B | B | |

Test Example 4-3. Substrate: Polyethylene Terephthalate (PET)

Evaluation was performed in the same manner as in Test Example 4-1, except that polyethylene terephthalate (PET) resin plates were used as the base material. Table 5 shows the results.

Table 5

| | Example 2 | Example 4 | Example 5 | Example 6 |
|---|---|---|---|---|
| Base material: PET | A | A | A | A |
| | Example 7 | Example 8 | Comparative Example 1 | Comparative Example 5 |
| Base material: PET | A | A | B | B |

Test Example 4-4. Base Material: Polymethyl methacrylate (PMMA)

Evaluation was performed in the same manner as in Test Example 4-1, except that polymethyl methacrylate (PMMA) resin plates were used as the base material. Table 6 shows the results.

Table 6

| | Example 2 | Example 4 | Example 5 | Example 6 | Example 7 | Example 8 | Comparative Example 1 |
|---|---|---|---|---|---|---|---|
| Base material: PMMA | A | A | A | A | A | A | B |

Test Example 4-5. Base Material: Polyvinyl Chloride (PVC)

Evaluation was performed in the same manner as in Test Example 4-1, except that polyvinyl chloride (PVC) resin plates were used as the substrate. Table 7 shows the results.

Table 7

| | Example 2 | Example 4 | Example 5 | Example 6 | Example 7 | Example 8 | Comparative Example 1 |
|---|---|---|---|---|---|---|---|
| Base material: PVC | A | A | A | A | A | A | B |

Test Example 4-6. Base Material: Polycarbonate (PC)

Evaluation was performed in the same manner as in Test Example 4-1, except that polycarbonate (PC) resin plates were used as the base material. Table 8 shows the results.

Table 8

| | Example 2 | Example 4 | Example 5 | Example 6 | Example 7 | Example 8 | Comparative Example 1 |
|---|---|---|---|---|---|---|---|
| Base material: PC | A | A | A | A | A | A | B |

Test Example 4-7. Substrate: stainless steel (SUS)

Evaluation was performed in the same manner as in Test Example 4-1, except that stainless steel (SUS) plates were used as the base material. Table 9 shows the results.

Table 9

| | Example 2 | Example 4 | Example 5 | Example 6 | Example 7 | Example 8 | Comparative Example 1 |
|---|---|---|---|---|---|---|---|
| Base material: SUS | A | A | A | A | A | A | B |

Test Example 5: Elution Test of Bulk Polymer

Each of the polymers obtained in the Examples and the Comparative Examples was placed into a 15-ml centrifuge tube. After 10 ml of water was added to the tube, shaking was performed using a shaker at room temperature for 24 hours. The resulting mixture was then centrifuged (at 10,000 rpm for 10 minutes) to precipitate each polymer. The supernatant other than the precipitate was removed by decantation to measure the weight change of the polymer. The weight reduction percentage was defined as the elution rate. Table 10 below shows the results.

Test Example 6: Elution Test of Coating Film

5 cm × 12.5 cm soda glass (0.7 mm in thickness) was coated with each of the solutions of the polymers obtained in the Examples and the Comparative Examples (to a wet film thickness of 50 μm) using a bar coater, and then dried in a heated-air oven at 90°C for 1 hour to obtain a coated base material. The obtained coated base material was immersed in 500 ml of water, and allowed to stand at room temperature for 20 hours. After the coated base material was removed from the water and dried, the weight change was measured. The weight reduction percentage was defined as the elution rate. Table 10 below shows the results.

Table 10

| | | Example 2 | Comparative Example 1 | Comparative Example 3 |
|---|---|---|---|---|
| Elution rate (%) | Bulk | 0 | 1 | 40 |
| | Coating film | 1 | 3 | 97 |

The results show the following. Since the copolymer of Comparative Example 5, which is a random copolymer of several types of monomers, had no intermediate water as shown in Table 1, antithrombogenicity was not imparted and the copolymer also had insufficient adhesion as shown in Table 3. In contrast, the block copolymers obtained in Examples 1 to 8 had intermediate water, and further had excellent adhesion. The polymer obtained in Comparative Example 1 had intermediate water as shown in Table 1, and short-term antithrombogenicity was imparted as shown in Table 2. However due to its poor adhesion as shown in Table 3, the polymer of Comparative Example 1 fails to solve the problem such that antithrombogenicity does not last for a long period of time.

## Claims

**1.** A block copolymer composition comprising intermediate water and a block copolymer, wherein the intermediate water is water in a state in which an exothermic peak based on a low-temperature crystallization of water is observed at or below 0 °C in an elevated temperature process from -100 °C in measurement using a differential scanning calorimeter (DSC),

the block copolymer comprising an A block and a B block, wherein the A block is capable of containing intermediate water, and the B block is more hydrophobic than the A block,
the A block is a polymer block comprising a vinyl monomer-derived structural unit capable of containing intermediate water, in which the vinyl monomer is at least one selected from vinyl monomers represented by the following formulae (1) and (3):

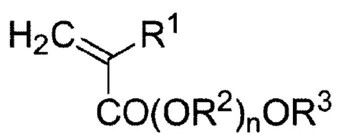
(1)

(wherein $R^1$ is a hydrogen atom,

$R^2$ is alkylene having 2 or 3 carbon atoms,
$R^3$ is methyl or ethyl, and
n is 1);

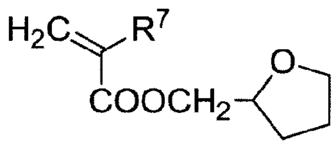
(3)

(wherein $R^7$ is a hydrogen atom or methyl);

the B block is a polymer block comprising a vinyl monomer-derived structural unit, in which the vinyl monomer is at least one selected from the group consisting of (meth)acrylic acid alkyl esters, alicyclic alkyl esters of (meth)acrylic acid, (meth)acrylic acid aryl esters, N,N-disubstituted aminoalkyl (meth)acrylate, and aromatic vinyl monomers, and
the A block and the B block are present at a molar ratio (A:B) of 85:15 to 40:60.

**2.** The block copolymer composition according to claim 1, wherein the block copolymer has a weight average molecular weight (Mw) of 10,000 to 1,000,000.

**3.** The block copolymer composition according to claim 1 to 2, wherein the block copolymer has a molecular weight distribution (Mw/Mn) of 3.0 or less.

**4.** The block copolymer composition according to any one of claims 1 to 3, wherein the block copolymer is a living radical polymerization reaction product.

**5.** A block copolymer for use as an antithrombotic agent,

the block copolymer comprising an A block and a B block, wherein the A block is capable of containing intermediate water, and the B block is more hydrophobic than the A block,
wherein the intermediate water is water in a state in which an exothermic peak based on a low-temperature crystallization of water is observed at or below 0 °C in an elevated temperature process from -100 °C in measurement using a differential scanning calorimeter (DSC),
the A block is a polymer block comprising a vinyl monomer-derived structural unit, in which the vinyl monomer is at least one selected from vinyl monomers represented by the following formulae (1) and (3):

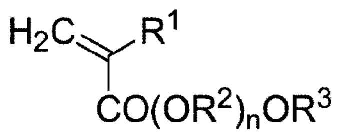
(1)

(wherein $R^1$ is a hydrogen atom,

$R^2$ is alkylene having 2 or 3 carbon atoms,
$R^3$ is methyl or ethyl, and
n is 1);

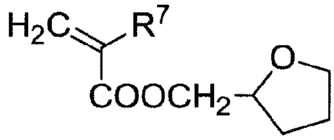
(3)

(wherein $R^7$ is a hydrogen atom or methyl);
the B block is a polymer block comprising a vinyl monomer-derived structural unit, in which the vinyl monomer is at least one selected from the group consisting of (meth)acrylic acid alkyl esters, alicyclic alkyl esters of (meth)acrylic acid, (meth)acrylic acid aryl esters, N,N-disubstituted aminoalkyl (meth)acrylate, and aromatic vinyl monomers, and
the A block and the B block are present at a molar ratio (A:B) of 85:15 to 40:60.

6. The block copolymer for use according to claim 5, wherein the block copolymer is present as a coating.

7. The block copolymer for use according to claim 6, wherein the coating is present on a base material.

**Patentansprüche**

1. Eine Blockcopolymer-Zusammensetzung, umfassend intermediäres Wasser und ein Blockcopolymer, wobei das intermediäre Wasser Wasser in einem Zustand ist, bei dem ein exothermer Peak basierend auf einer Kristallisation von Wasser bei niedriger Temperatur bei oder unterhalb von 0°C in einem Verfahren mit erhöhter Temperatur von -100°C bei der Messung unter Verwendung eines Differentialscanningkalorimeters (DSC) beobachtet wird,
wobei das Blockcopolymer einen A-Block und einen B-Block umfasst, wobei der A-Block intermediäres Wasser enthalten kann und der B-Block hydrophober ist als der A-Block, wobei der A-Block ein Polymerblock ist, der eine von Vinylmonomer abgeleitete Struktureinheit umfasst, die intermediäres Wasser enthalten kann, wobei das Vinylmonomer mindestens eines ist, das aus Vinylmonomeren, dargestellt durch die folgenden Formeln (1) und (3), ausgewählt ist:

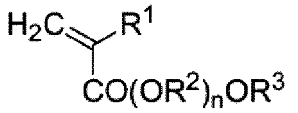
(1)

(wobei $R^1$ ein Wasserstoffatom ist,

$R^2$ Alkylen mit 2 oder 3 Kohlenstoffatomen ist,
$R^3$ Methyl oder Ethyl ist und
n gleich 1 ist),

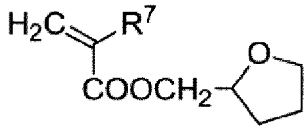
(3)

(wobei $R^7$ ein Wasserstoffatom oder Methyl ist);

wobei der B-Block ein Polymerblock ist, der eine von Vinylmonomer abgeleitete Struktureinheit umfasst, wobei das Vinylmonomer mindestens eines ist, das aus der Gruppe bestehend aus (Meth)acrylsäurealkylestern, alicyclischen Alkylestern von (Meth)acrylsäure, (Meth)acrylsäurearylestern, N,N-disubstituiertem Aminoalkyl(meth)acrylat und aromatischen Vinylmonomeren, ausgewählt ist und
der A-Block und der B-Block in einem Molverhältnis (A:B) von 85:15 bis 40:60 vorliegen.

2. Die Blockcopolymer-Zusammensetzung nach Anspruch 1, wobei das Blockcopolymer ein Gewichtsmittel des Molekulargewichts (Mw) von 10.000 bis 1.000.000 aufweist.

3. Die Blockcopolymer-Zusammensetzung nach Anspruch 1 bis 2, wobei das Blockcopolymer eine Molekulargewichtsverteilung (Mw/Mn) von 3,0 oder weniger aufweist.

4. Die Blockcopolymer-Zusammensetzung nach einem der Ansprüche 1 bis 3, wobei das Blockcopolymer ein Produkt einer lebenden radikalischen Polymerisationsreaktion ist.

5. Ein Blockcopolymer zur Verwendung als ein Antithrombosemittel,

wobei das Blockcopolymer einen A-Block und einen B-Block umfasst, wobei der A-Block intermediäres Wasser enthalten kann und der B-Block hydrophober ist als der A-Block, wobei das intermediäre Wasser Wasser in einem Zustand ist, bei dem ein exothermer Peak basierend auf einer Kristallisation von Wasser bei niedriger Temperatur bei oder unterhalb 0°C in einem Verfahren mit erhöhter Temperatur von -100°C bei der Messung unter Verwendung eines Differentialscanningkalorimeters (DSC) beobachtet wird,

wobei der A-Block ein Polymerblock ist, der eine von Vinylmonomer abgeleitete Struktureinheit umfasst, wobei das Vinylmonomer mindestens eines ist, das aus Vinylmonomeren, dargestellt durch die folgenden Formeln (1) und (3), ausgewählt ist:

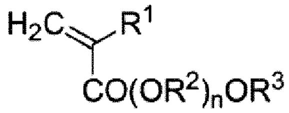
(1)

(wobei $R^1$ ein Wasserstoffatom ist,

$R^2$ Alkylen mit 2 oder 3 Kohlenstoffatomen ist,
$R^3$ Methyl oder Ethyl ist und
n gleich 1 ist),

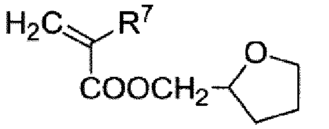
(3)

(wobei $R^7$ ein Wasserstoffatom oder Methyl ist);
wobei der B-Block ein Polymerblock ist, der eine von Vinylmonomer abgeleitete Struktureinheit umfasst, wobei das Vinylmonomer mindestens eines ist, das aus der Gruppe bestehend aus (Meth)acrylsäurealkylestern, alicyclischen Alkylestern von (Meth)acrylsäure, (Meth)acrylsäurearylestern, N,N-disubstituiertem Aminoalkyl(meth)acrylat und aromatischen Vinylmonomeren, ausgewählt ist und
der A-Block und der B-Block in einem Molverhältnis (A:B) von 85:15 bis 40:60 vorliegen.

6. Das Blockcopolymer zur Verwendung nach Anspruch 5, wobei das Blockcopolymer als eine Beschichtung vorliegt.

7. Das Blockcopolymer zur Verwendung nach Anspruch 6, wobei die Beschichtung auf einem Basismaterial vorliegt.

## Revendications

1. Composition de copolymère bloc comprenant de l'eau intermédiaire et un copolymère bloc, dans laquelle l'eau intermédiaire est de l'eau dans un état dans lequel une crête exothermique sur la base d'une cristallisation de l'eau à basse température est observée à 0 °C ou en-deçà lors d'un processus à température élevée à partir de -100 °C lors d'une mesure en utilisant un calorimètre à balayage différentiel (DSC) ;

le copolymère bloc comprenant un bloc A et un bloc B, dans laquelle le bloc A est capable de contenir de l'eau intermédiaire et le bloc B est davantage hydrophobe que le bloc A ;
le bloc A est un bloc de polymère qui comprend une unité structurelle qui est dérivée d'un monomère de vinyle et qui est capable de contenir de l'eau intermédiaire, dans laquelle le monomère de vinyle est au moins un monomère de vinyle qui est sélectionné parmi les monomères de vinyle qui sont représentés par les formules (1) et (3) qui suivent :

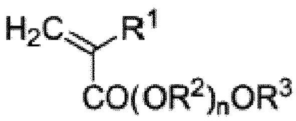
(1)

(dans laquelle :

$R^1$ est un atome d'hydrogène ;
$R^2$ est un alkylène qui comporte 2 ou 3 atomes de carbone ;
$R^3$ est un méthyle ou un éthyle ; et
n est 1) ;

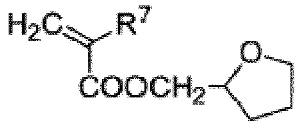

(3)

(dans laquelle $R^7$ est un atome d'hydrogène ou un méthyle) ;
le bloc B est un bloc de polymère qui comprend une unité structurelle qui est dérivée d'un monomère de vinyle, dans laquelle le monomère de vinyle est au moins un monomère de vinyle qui est sélectionné parmi le groupe qui est constitué par les esters alkyliques d'acide (méth)acrylique, les esters alkyliques alicycliques d'acide (méth)acrylique, les esters aryliques d'acide (méth)acrylique, un (méth)acrylate aminoalkylique N,N-disubstitué et les monomères de vinyle aromatiques ; et
le bloc A et le bloc B sont présents selon un rapport molaire (A:B) de 85:15 à 40:60.

**2.** Composition de copolymère bloc selon la revendication 1, dans laquelle le copolymère bloc présente un poids moléculaire moyen en poids (Mw) de 10 000 à 1 000 000.

**3.** Composition de copolymère bloc selon la revendication 1 à 2, dans laquelle le copolymère bloc présente une distribution de poids moléculaire (Mw/Mn) de 3,0 ou moins.

**4.** Composition de copolymère bloc selon l'une quelconque des revendications 1 à 3, dans laquelle le copolymère bloc est un produit de réaction de polymérisation radicalaire vivante.

**5.** Copolymère bloc pour une utilisation en tant qu'agent antithrombotique,

le copolymère bloc comprenant un bloc A et un bloc B, dans lequel le bloc A est capable de contenir de l'eau intermédiaire et le bloc B est davantage hydrophobe que le bloc A ;
dans lequel l'eau intermédiaire est de l'eau dans un état dans lequel une crête exothermique sur la base d'une cristallisation de l'eau à basse température est observée à 0 °C ou en-deçà lors d'un processus à température élevée à partir de -100 °C lors d'une mesure en utilisant un calorimètre à balayage différentiel (DSC) ;
le bloc A est un bloc de polymère qui comprend une unité structurelle qui est dérivée d'un monomère de vinyle, dans lequel le monomère de vinyle est au moins un monomère de vinyle qui est sélectionné parmi les monomères de vinyle qui sont représentés par les formules (1) et (3) qui suivent :

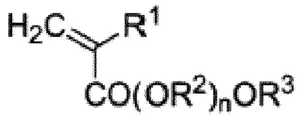

(1)

(dans laquelle :

$R^1$ est un atome d'hydrogène ;
$R^2$ est un alkylène qui comporte 2 ou 3 atomes de carbone ;
$R^3$ est un méthyle ou un éthyle ; et
n est 1) ;

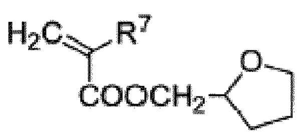

(3)

(dans laquelle $R^7$ est un atome d'hydrogène ou un méthyle) ;
le bloc B est un bloc de polymère qui comprend une unité structurelle qui est dérivée d'un monomère de vinyle, dans lequel le monomère de vinyle est au moins un monomère de vinyle qui est sélectionné parmi le groupe qui est constitué par les esters alkyliques d'acide (méth)acrylique, les esters alkyliques alicycliques d'acide (méth)acrylique, les esters aryliques d'acide (méth)acrylique, un (méth)acrylate aminoalkylique N,N-disubstitué et les monomères de vinyle aromatiques ; et

le bloc A et le bloc B sont présents selon un rapport molaire (A:B) de 85:15 à 40:60.

6. Copolymère bloc pour une utilisation selon la revendication 5, dans lequel le copolymère bloc est présent en tant que revêtement.

7. Copolymère bloc pour une utilisation selon la revendication 6, dans lequel le revêtement est présent sur un matériau de base.

Fig. 1

Example 1

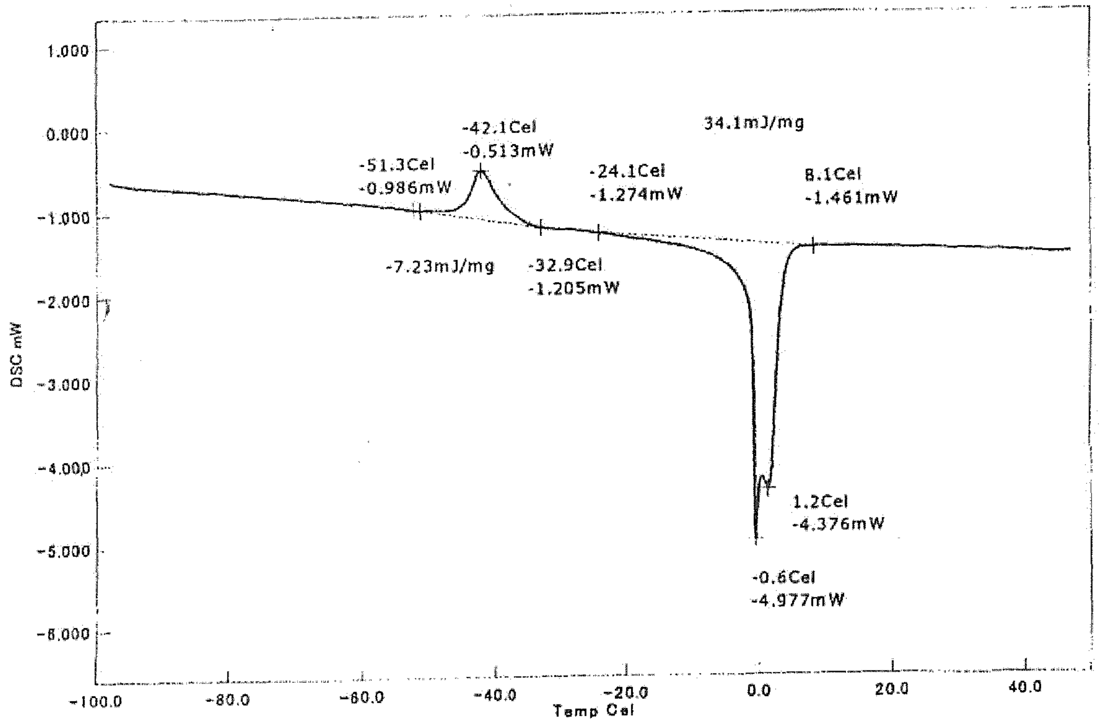


Fig. 2

Comparative Example 4

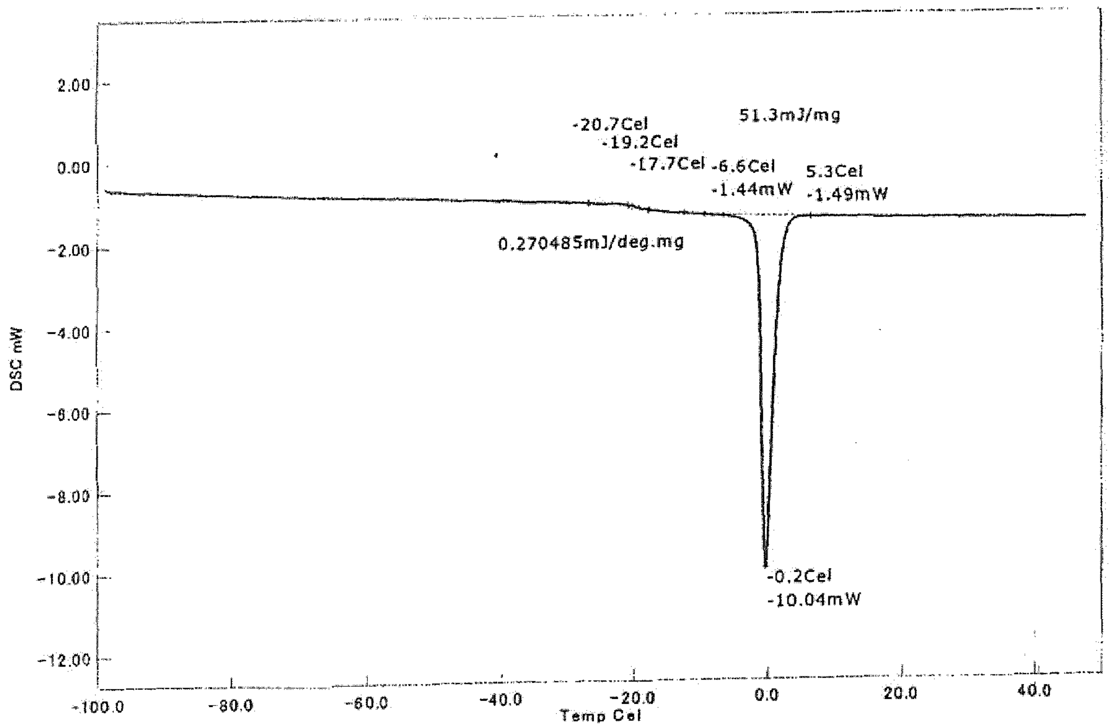

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013056146 A
- EP 2071584 A
- JP 2006117713 A
- WO 200414848 A
- WO 200414962 A
- JP 2004161954 A


### Non-patent literature cited in the description


- *Langmuir,* 2008, vol. 24 (10), 5527-5533
- *Polymer Journal,* 2013, vol. 45 (7), 701-710